# EUROPEAN PATENT SPECIFICATION

(11) **EP 0 613 400 B1**
(45) Date of publication and mention of the grant of the patent: **16.04.1997**
(21) Application number: 93901501.2
(22) Date of filing: 20.11.1992
(51) Int. Cl.: B01L 3/00, G01N 33/53

(54) **TEST DEVICE COMPRISING A PLATE CONTAINING A MULTIPLICITY OF WELLS WITH AN ASSOCIATED METERING DEVICE, AS WELL AS A KIT WHICH COMPRISES THESE DEVICES AND USE OF THE DEVICES**
TESTVORRICHTUNG, EINE MEHRLOCHPLATTE UND EINE DAZUGEORDNETE DOSIERVORRICHTUNG UMFASSEND, SOWIE EIN TESTSATZ MIT DIESEN GERÄTEN UND EINE ANVENDUNG DAVON
DISPOSITIF D'ANALYSE COMPORTANT UNE PLAQUE CONTENANT UNE PLURALITE D'ALVEOLES ASSOCIEES A UN DISPOSITIF DOSEUR, KIT COMPORTANT DE TELS DISPOSITIFS, ET UTILISATION DE CEUX-CI

(30) Priority: 21.11.1991 NL 9101953
(43) Date of publication of application: 07.09.1994
(73) Proprietor: PEPSCAN SYSTEMS B.V., 8219 PH Lelystad (NL)
(72) Inventor: PUIJK, Wouter, Cornelis, NL-8243 VZ Lelystad (NL); LIGTVOET, Gerard, Johannes, NL-2321 EP Leiden (NL); MELOEN, Robert, Hans, NL-8231 AP Lelystad (NL)
(74) Representative: de Bruijn, Leendert C.
(86) International application number: NL9200213
(87) International publication number: WO9309872

(56) References cited:
- EP-A- 0 018 435
- EP-A- 0 347 579
- EP-A- 0 542 422
- WO-A-91/06859
- DD-A- 107 783
- FR-A- 2 383 442
- US-A- 4 200 613
- US-A- 4 735 778

## Description

The present invention relates to a test device comprising a plate containing a multiplicity of wells. The invention also relates to a metering device suitable for the simultaneous introduction of equal volumes of reagent into different wells of the test device. In addition, the invention relates to a method for carrying out a (bio- and/or immuno) chemical test using the test device and/or the metering device and the invention is aimed at a kit which comprises the test device and the metering device.

A test device comprising a plate containing a multiplicity of wells has already been known for years in the form of the so-called microtitre plate. The known microtitre plate is of the order of 12.5 cm × 8.0 cm in size and comprises 96 wells. The diameter of each well is about 0.6 cm and the depth of each well is about 1.0 cm, so that each well can contain at most a 250 µl sample. The wells are separated by material barriers with a width of approximately 2.0 mm.

The known microtitre plate is used when carrying out diverse bio- and/or immunochemical tests. In tests of this type, photometric detection is frequently used. A very well-known example of such a test is the ELISA. In the case of photometric determinations, the bottom of a well must be uniformly covered with a layer of the sample to be analysed in order to obtain reliable results. Furthermore, this layer must have a thickness which is at least such that detectable absorption occurs. In practice, this generally implies that samples are used which have at least a volume of 50 µl.

In WO-91/06859 an assay method is described for detecting the response or activation of cells including lymphocytes. A method for detecting immunological sensitisation in a subject which involves the introduction of a cell activating substance which causes an enzyme of the cells to become available for reaction and the measurement of the enzymatic reaction using a substrate which generates a detectable product and a kit for performing such assays are disclosed. Use of a sample volume of 30 µl is mentioned as a sample of preference to be used in a microtitre plate well with a diameter of 6 mm. A reaction vessel is claimed with a diameter of 6 mm and a height of 0,5-6 mm with a preferred height of 1-2 mm. In the cited document the vessel is designed to optimize physiological control of the reaction allowing adequate exchange of gasses such as oxygen and CO₂, sufficiently rapid pH equilibration, adequate diffusion of molecules and dissipation of heat. The reaction vessel of the cited document is concerned with the ratio of the reaction volume and the surface area of the reaction sample. The cited document does not disclose any problems that can be encountered when rinsing small reaction vessels in processes, such as ELISA or PEPSCAN.

In EP-A-0 542 422 published on 19.05.93 with a priority date of 12.11.91, a document according to Article 54(3) and (4) EPC designating CH, DE, FR, GB and LI a well having a displaced volume of 20 µl is disclosed. A well having a smaller volume is not disclosed. A metering device for introducing equal volumes of reagents into different wells of a test device comprising a microtitre plate containing a multiplicity of wells is described, said device comprising 864 projections. The cited document described a multiwell microtitre tray which is useful for simultaneously processing a high volume of minute liquid samples. Such a multiwell microtitre tray is useful with robotics which function in accordance with existing standards of the industry. The top of the microtitre tray is formed with 864 wells which are arranged in a rectangular array that is 36 by 24. The system for using the multiwell microtitre tray includes a support structure which has a heat exchanger that is engageable with the microtitre tray.

Since bio- and/or immunochemical tests frequently involve large numbers of tests on samples (such as blood and sera) which have to be obtained from test persons and/or animals, there is a need to use as little sample as possible per test.

However, the sample can be diluted to only a limited degree since the component to be analysed must also be present in the well in a certain minimum concentration in order to obtain measurable absorption. This is because, according to Lambert-Beers' law, the light intensity is dependent on the concentration and the absorption coefficient of the component to be analysed and also on the distance the light has to travel through the sample to be measured. In practice, this implies the use of about 12.5 ml of reagents per microtitre plate. In such cases there is an appreciable need to reduce the amount of sample to be used.

Another frequent use of the current microtitre plate is in the synthesis of peptides. In such syntheses peptides containing different amino acid sequences can be synthesised. This can be carried out, for example, with a view to determining the location of an epitope of a protein for a specific antibody. To this end, peptides containing amino acid sequences corresponding to a fragment of the protein to be studied are synthesised separately. The synthesis can be carried out in such a way that each peptide in part contains the amino acid sequence of another peptide. It is even possible to carry out the synthesis in such a way that only one amino acid does not overlap. It is also possible to produce a series of short sections, for example hexapeptides which overlap with the exception of one amino acid. A determination is then carried out to establish with which peptide antibody binding takes place. Peptides with which antibody binding takes place contain an epitope.

In the first instance, peptide synthesis was carried out by adding the amino acid to be coupled to the well of the known microtitre plate in which the peptide had to be synthesised, then coupling the desired amino acid to the growing peptide chain, subsequently washing the well to remove any unreacted amino acid and repeating the procedure with the next amino acid.

However, with this method problems were experienced in rinsing the wells and, therefore, a method of peptide synthesis was adopted in which small polyethylene rods are used as supports for the growing peptide chains. This method is described by Geysen, H.M., Meloen, R.H. and Barteling, S.J. beschreven in Proc. Natl. Acad. Sci. USA, Vol. 81 (July 1984) pp. 3998-4002. In this article, a method is described for the simultaneous synthesis of hundreds of peptides on a solid support with adequate purity for carrying out an ELISA. Interaction of the peptides with antibodies can be detected simply, without removing the peptides from the support. Consequently, it becomes possible to determine an immunogenic epitope with a good resolution. This method is termed the PEPSCAN.

With this method, the growing peptide chains are allowed to adhere to polyethylene rods (having a diameter of 4 mm and a length of 40 mm) and the reactions required for peptide synthesis are then carried out using the ends of the support rods. To this end, the polyethylene rods are first immersed in a 6 percent solution of acrylic acid in water and subjected to γ-radiation. For the subsequent reactions, the ends of the rods are then brought into contact with a Teflon plate containing a matrix of wells corresponding to the location of the rods (the known microtitre plate). The conventional methods for peptide chemistry in the solid phase can be used here, for example for coupling N^{α}-t-butyloxycarbonyl-L-lysine methyl ester to polyethylene/polyacrylic acid via the N^{α}-amino group of the side chain. [(Erickson, B.W. and Merrifield, R.B. (1976) in The Proteins, Eds. Neurath, H & Hill, R.L. (Academic, New York), Vol. 2, pp. 255-527) and (Meienhofer, J. (1973) in Hormonal Proteins and Peptides, Ed. Li, C.H., (Academic, New York), Vol. 2, pp. 45-267)]. After removing the t-butyloxycarbonyl group, t-butyloxycarbonyl-L-alanine can be coupled, a peptide-like spacer being formed. The desired amino acids can be coupled successively and, following the final desired coupling reaction and after removal of the protecting t-butyloxycarbonyl group, the terminal amino acid can be acetylated using acetic anhydride in dimethylformamide/triethylamine. All coupling reactions carried out with N,N-dicyclohexylcarbodiimide can be carried out in dimethylformamide in the presence of N-hydroxybenzotriazole. Any protective groups in side chains of amino acids used in the peptide synthesis can also be removed. Before the synthesised peptides are examined further, for example by means of ELISA, the rods can be washed with a phosphate-buffered saline solution.

Another use of peptide synthesis takes place if one or more amino acids of a known epitope are changed in order to determine which other sequences are able to function as an epitope and/or in order to determine which amino acids are essential for the epitope action. A method of this type is described by Geysen, H.M., Meloen, R.H. and Barteling, S.J. in Proc. Natl. Acad. Sci. USA, Vol. 82 (January 1985) pp. 178-182.

As such methods frequently comprise a large number of syntheses and thus also the use of large amounts of reagents, which reagents, moreover, are frequently expensive, there is also a need, with a view to reducing costs, to use sample amounts which are as small as possible. Possibilities have therefore been sought for miniaturisation of such peptide syntheses.

A method for miniaturised peptide synthesis has recently been described in an article by Fodor, S.P.A. et al. (Science, (15 February 1991) pp. 767-773). In this method light is used to control the simultaneous synthesis of a large number of different chemical compounds. Synthesis takes place on a solid support, such as a glass plate. The support is aminated by treatment with 0.1 % aminopropyltriethoxysilane in 95 % ethanol. Here, a light-sensitive protective group is then introduced, said protective group disappearing following irradiation with light and giving a reactive site to which a building block, such as an amino acid, can be coupled. The pattern in which exposure to light or other forms of energy takes place (for example via a mask) determines which areas of the support are activated for chemical coupling. The entire surface is brought into contact with the building block to be coupled (said building block also being provided with a light-sensitive protective group). A coupling reaction will occur only at sites where the light in the previous step has given rise to activation. The substrate is then exposed through another mask, so that a subsequent building block can be incorporated in the desired site. The pattern of the mask and the sequence of the reagents determine the sequences of the peptides formed. A high degree of miniaturisation can be achieved in this way. For example, it is possible to synthesise 40,000 different peptides on 1 cm².

However, this method has a number of disadvantages. The removal of the protective light-sensitive group (nitroveratryloxycarbonyl is named in the article) takes place by irradiation for 20 minutes with a mercury lamp having a power of 12 mW/cm³. This will result in a very long synthesis time in the case of the synthesis of longer peptides. Furthermore, a different mask will have to be used for each addition step and a different set of masks will have to be used for each series of peptides.

Moreover, only one building block can be added in each addition step because the various peptides to be synthesised are not spatially separated. It is obvious that mixing of reagents would otherwise take place and, thus, undesired products would also form. This method is therefore very laborious, especially for the synthesis of peptides which differ not only in respect of length but also in respect of sequence.

The authors of the article themselves also touch on the problem of the reliability of the synthesis. Deletions can occur as a consequence of incomplete removal of the protective group, following irradiation with light. The net coupling percentage is 85-95 %. Furthermore, when changing masks, a certain overlap between the diverse synthesis regions will take place because of light diffraction, internal reflection and scattering. Consequently, compounds will be formed in regions which are considered to be dark, as a result of which undesired insertion of a specific amino acid can take place.

In FR-A-2 383 442 (Institut Pasteur) a solution for miniaturisation problems is given. The solution implies carrying out miniaturisation reactions with additional means such as centrifugation and an additional receptacle for the actual reaction. The volume of the micro cuvet described in the cited document could be in the order of a few micro liters however the micro cuvet is merely destined for measurement of a sample that has already undergone the reaction steps required in a different much larger receptacle. It is obvious from the teaching of the French document that the micro cuvet is not suitable for carrying out a reaction. A rather elaborate construction is shown wherein an intermediate receptacle is required along with a centrifugation step to carry out a reaction after which the reaction mixture is subsidently removed to the micro cuvet. In particular as a reaction such as a PEPSCAN involves a large number of reaction steps entailing a number of rinsing steps the method as taught in the cited document would not be acceptable for carrying out a miniaturised PEPSCAN.

The present invention relates to a test device which solves the miniaturisation problems described above and is suitable for use for bio- and/or immunochemical tests such as ELISA and tests in which peptide syntheses are used, for example the PEPSCAN as described above.

The present invention relates to a test device which comprises a plate containing a multiplicity of wells, which is characterised in that the wells have a volume within the range of 0.1-20 µl and that the diameter of the wells is 1.0-4.0 mm. The dimensions of the wells will be chosen depending on the price and availability of the samples and reagents to be used. In general, wells which are as small as possible will be preferred and, therefore, the present invention preferably relates to a test device in which the wells have a volume within the range of 0.1-5 µl.

Entirely contrary to expectations, it has now been found that making the wells smaller has no adverse consequences for the efficient rinsability thereof. It has been found that the rinsing times required in order to obtain good rinsing are shortest if the ratio between the depth of the wells and the diameter thereof is less than 1:1. Therefore, a test device comprising a plate containing a multiplicity of wells, characterised in that the wells have a volume within the range of 0.1-20 µl and that the diameter of the wells is 1.0-4.0 mm, further having a ratio between the depth of the wells and the diameter thereof below 1:1, is very suitable. A test device according to the invention for which the ratio between the depth of the wells and the diameter thereof is less than 2:3 is preferred.

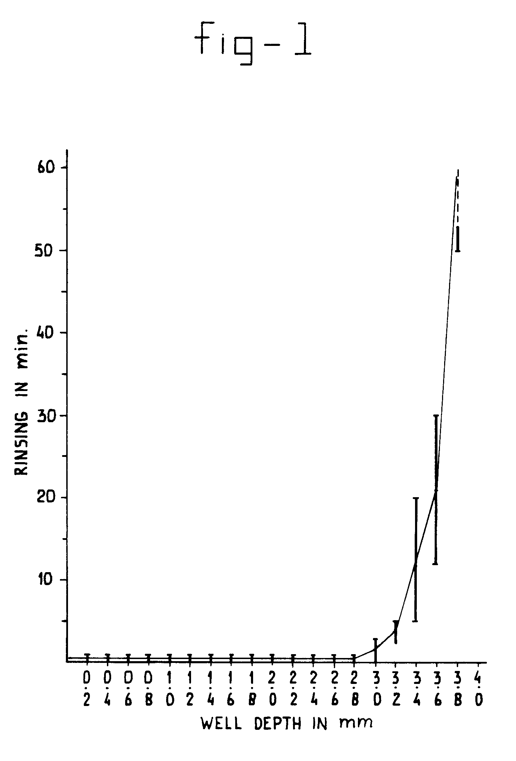

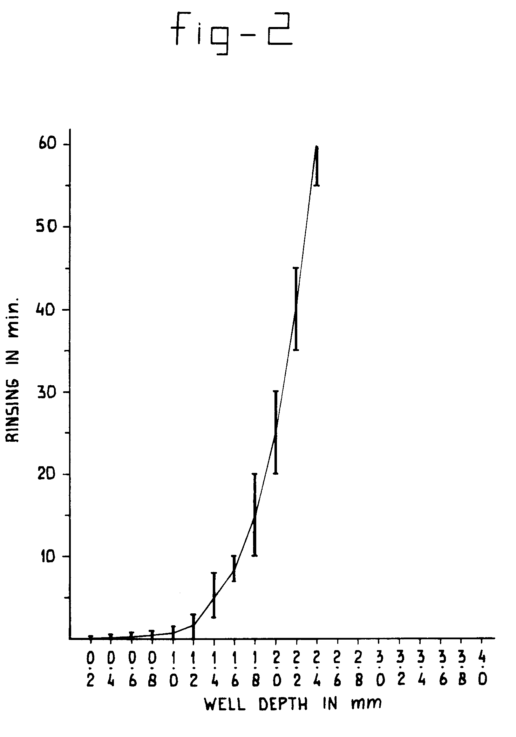

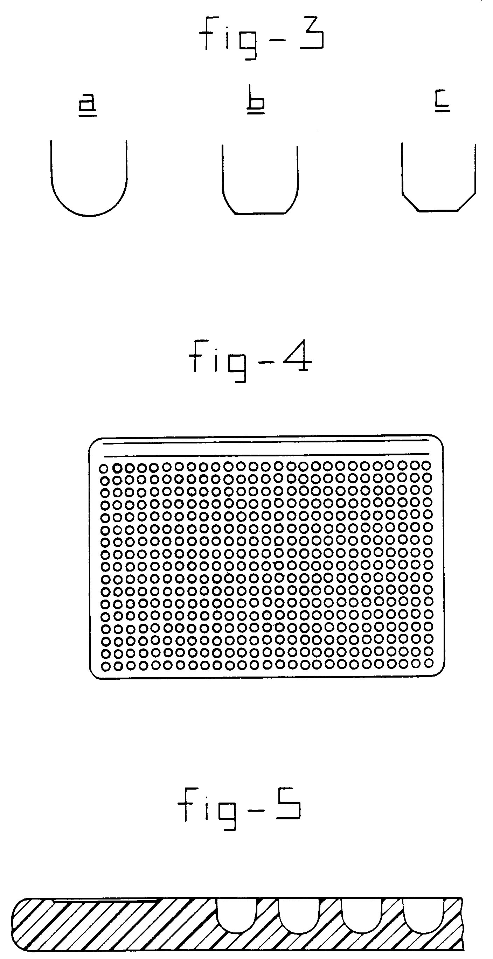

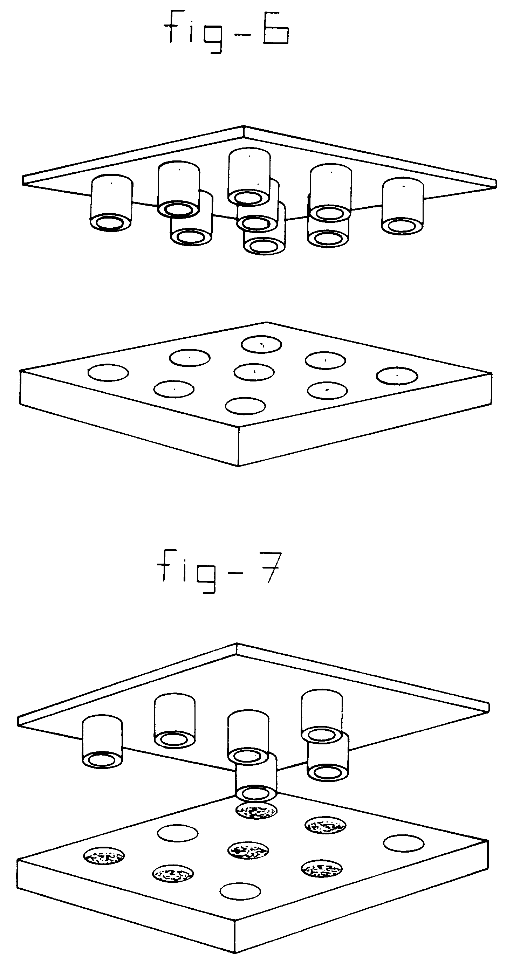

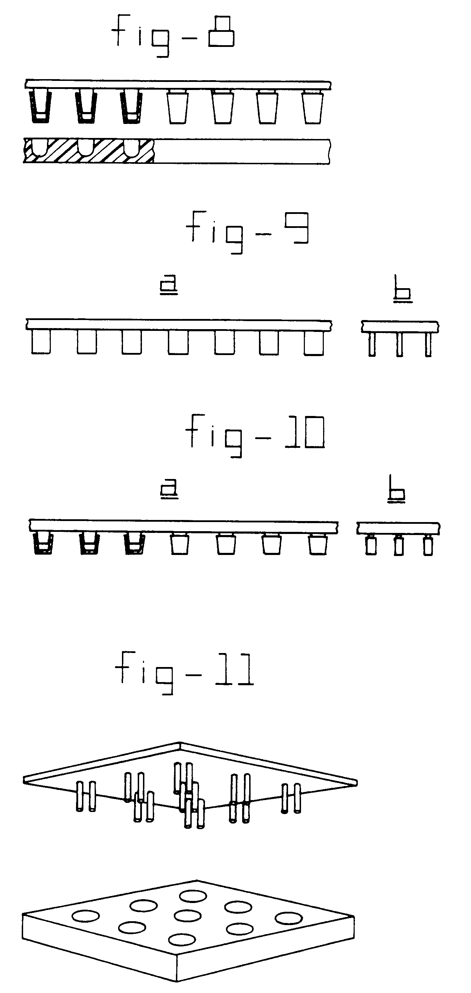

Figures 1 and 2 show the results of tests in which the rinsability of various test devices was investigated. The test devices had wells of equal diameter (2 mm) but of different depths. The rinsability was investigated on a shaking machine at speeds of, 47 and 40, respectively. The depth of the well in mm is plotted against the time in minutes needed to properly rinse the well.

The invention is preferably aimed at a test device in which wells with a diameter of 1.0-2.0 mm are preferred. The choice of the dimensions of the wells will depend on the desired specific test for which the test device is to be used. The smaller the diameter, the smaller the required volume of the sample.

In connection with the desired good rinsability, it is also preferred that the wells have a shape such that a vertical cross section of the wells is essentially U-shaped, the transition between legs and base of the U being gradual. Preferably there are no sharp angles in the well.

A number of suitable shapes of wells are shown in Figure 3.

The U shape in which the angle between base and legs is perpendicular is preferred for photometric determinations in which measurement is carried out under and through the plate.

The test device according to the invention will preferably be a plate containing wells separated by material barriers with a width of 1.0-5.0 mm, preferably by material barriers with a width between 1.0 and 2.0 mm. The material barriers must be sufficiently wide to prevent reagents flowing over from one well to another. Specifically, the material barriers must be sufficiently wide when DMF is used as solvent, as is frequently the case in peptide synthesis. This is because DMF is known to have a high creeping capacity.

The test device according to the invention will in a suitable embodiment comprise a plate containing 5-20 wells per cm², preferably 10-15 wells per cm².

Furthermore, the test device will in a suitable embodiment comprise material to which peptides, proteins and other biochemical molecules, such as hormones and polysaccharides, are able to adhere. For test devices suitable for tests with peptide synthesis, such material will preferably be material to which peptides and proteins are able to adhere, such as polyethylene or polystyrene.

Other suitable examples of materials which can be used in a test device according to the invention are polypropylene and polycarbonate.

The choice of the material for the test device will also depend on the reagents to be used in such a test device and on the detection method. In the case of photometric analysis through the bottom of the test device, for example, at least the bottom of the wells will have to be composed of transparent material. In the case of tests where DMF is used as solvent, it will not be possible to use a polystyrene test device because DMF is too aggressive.

A preferred embodiment of the test device will be provided with a means for recording information, for example a bar code or a magnetic strip. The test device can be provided with data relating to the test it is intended to carry out, or which has been carried out, such data for example relating to reagents or samples which have been used. The test device can also be provided with markings which indicate the coordinations of wells in the test device.

Figure 4 shows a top view of an example of one embodiment of the test device according to the invention.

A cross section is shown in Figure 5.

As already mentioned in the preamble, the present invention is also aimed at a method for carrying out (bio- and/or immuno)chemical tests, in which a test device according to the invention is used. In general, a test device according to the invention can be used in the same tests as the known microtitre plate. It is now possible to carry out existing methods using much smaller amounts of sample and reagents; so-called mini-tests are now possible. It is possible to reduce the amount of sample used by a factor of one hundred. It is now possible to use 2.5 µl instead of 250 µl samples per well. Use of the test device according to the invention now makes mass screening of population groups much more attractive because much less blood is required from the donor and much smaller quantities of reagents are required.

Another great advantage of the miniaturisation of the methods by use of the test device according to the invention lies in the fact that the existing chemistry does not have to be modified. In this context consider the great advantage, for example, in the case of automated processes, such as the PEPSCAN. The test device is particularly suitable for use in methods in which large numbers of samples have to be used. A mini-ELISA and mini-PEPSCAN in which a test device according to the invention is used are suitable examples of the method according to the present invention. The advantage of a mini-method according to the invention is that the test can be carried out with sample amounts of less than 20 µl. It is readily possible to use sample amounts of less than 5 µl in a mini-method according to the invention.

The present invention is also aimed at a metering device suitable for simultaneously introducing equal volumes of reagent into different wells in a test device according to the invention. A metering device according to the invention can be used in order to carry out as efficiently as possible immuno- and/or biochemical tests, said device being provided with projections having dimensions and mutual spacings such that individual projections can simultaneously be positioned in or above wells of a test device according to the invention. In this context, consideration is given to optional automation of certain methods according to the invention.

If a predetermined equal amount of reagent has to be introduced simultaneously into a number of wells, it is possible to use a metering device according to the invention. With a metering device of this type, all wells can simultaneously be provided with equal volumes of reagent if the position of the projections is such that this essentially corresponds to the position of the wells in the test device according to the invention.

One embodiment of the metering device and the test device is shown in Figure 6.

If not all, but only a certain number, of wells in the test device have to be filled, a metering device can be used which has projections which simultaneously can be positioned above or in the selected wells.

In Figure 7 the darker wells are the selected wells. The projections of the metering device are located on the metering device in such a way that they can simultaneously be positioned above or in the darker wells.

To this end, a metering device can advantageously be used in which the projections are fixed to the support or can be fixed in a pattern which corresponds to the pattern of the wells into which reagent has to be introduced.

Figure 8 shows an example of an embodiment of the metering device in which the projections can be fixed to a support.

The metering device can comprise projections which are fixed or can be fixed to a support in a way equivalent to the bristles of a brush (Figs. 6, 7 and 8).

A metering device in which the projections, like the teeth of a comb, are parallel to one another and are fixed (Fig. 9) or can be fixed (Fig. 10) at their tops to a support is also an embodiment of a metering device according to the invention which is very suitable. The number of projections can be less than or equal to the number of wells forming a row in the longitudinal direction of the test device. The number of projections can be less than or equal to the number of wells forming a row in the widthwise direction of the test device. The number of projections will depend on the pattern of wells of the test device into which reagent has to be introduced.

The projections of a metering device according to the invention can be integral with the support or can be detachable. The projections can be fitted on the support in such a way that the projections form a pattern which corresponds to the pattern of wells which have to be filled in the test device (see Fig. 7).

It is also possible to use a metering device in which more than one projection can be positioned above or in a well at the same time, if it is desired to simultaneously introduce more than one reagent unit, which is present on a projection, into a well.

Figure 11 shows a metering device in which two projections can be positioned simultaneously above or in each well.

Thus, the amount of reagent which is present on a projection can be taken as standard and metering devices can be used which have a group of projections above or in the well, depending on the ratio in which it is desired to introduce reagents into a well. A group will comprise the number of projections which corresponds to the number of desired reagent units.

In the case of a metering device according to the invention, the projections can be hollow, but they can also be solid or closed at the bottom. The latter two possibilities are to be preferred when working with very small amounts of sample and reagents, because it is then possible to work with drops of reagent.

The present invention is also aimed at a method for carrying out a (bio- and/or immuno-)chemical test using a metering device according to the invention, in which method
a) the projections of the metering device are provided with reagent, in such a way that essentially equal volumes of reagent are present on or in the individual projections of the metering device, and
b) the metering device is then positioned in or above wells of the test device according to the invention, which wells are intended to be provided with reagent, each individual projection being located in or above a well at the same time, and
c) essentially equal volumes of reagent are introduced into the individual wells of the test device, which wells it is intended to provide with reagent.

The invention also relates to a method of this type in which the projections of the metering device are simultaneously provided with reagent by immersing the projections in reagent.

The present invention is also aimed at a kit which comprises at least a test device and metering device according to the invention. Such a kit can comprise a number of metering devices in the various embodiments described above and can also comprise replaceable projections for such metering devices.

### Example 1

### Miniaturised peptide synthesis

Miniaturised synthesis of a complete tripeptide net (8000 different peptides) was carried out using test devices according to the invention. The test device used in this example resembled a credit card in size and was constructed so that it comprised 455 wells with a diameter of 2 mm and a maximum volume of 5 µl each. The test device was made of polyethylene. In order to make this solid carrier suitable for peptide synthesis the wells were treated via the method described by Geysen et al. (1984) previously mentioned. The carboxyl groups of the polyacrylic acid were provided with a NH₂-group via a linking reaction of a linker t-ButylOxyCarbonyl-HexaMethyleneDiAmine (BOC-HMDA) in the presence of N,N-DicyclohexylCarbodiimide and N-HydrOxyBenzotriazole (DDC/HOBt). All these linking reactions were carried out in DiMethylFormamide (DMF).

After removing the t-butyloxycarbonyl group with TriFluor Acetic acid (TFA) a mixture of all twenty L-amino acids linked using the same method as used for linking the linker was used.

The total volume used in the linking reaction amounted to 3 µl for each well. Pipetting the required small amounts was achieved completely automatically using a computer directed robot arm with a pipette installation (Hamilton MicroLab 2200). A special software program was written for this objective enabling two of test devices according to the invention described in this example to be filled per hour.

The linking time for each amino acid amounted to approximately 2 to 3 hours. The difference in linking time is caused by the reaction stopping when the reaction mixture has completely evaporated.

Subsequently after removal of the BOC-group with TFA the next amino acid was linked in the same manner after which the cycle was repeated twice more.

After the last linkage reaction and after removal of the BOC-group the terminal NH₂-group was acetylated with a mixture of acetic acid anhydride in DMF and TriEthylAmine in the ratio 2/5/1. The groups protecting the side groups were removed in a strong acid environment. In this instance BoriumTrisTrifluor acetic acid (BTT) in TFA (30 mg/ml) was used for two hours at room temperature.

The total structure of the peptide was as follows:
Ac-A₃-A₂-A₁-X-carrier,
wherein Ac represents an acetyl group, Aₓ represents a single amino acid and X represents the mixture of amino acids.

### Example 2

### ELISA

The test devices according to the invention were rinsed with phosphate buffered saline (PBS, 3x10 min) before incubation of the peptides with serum, after which the test devices according to the invention were precoated for 1 hour at 37°C with 10% horse serum/10% ovalbumine/1% Tween® 80 in PBS (SuperQ®) in order to prevent aspecific absorption of antibodies. The test devices according to the invention were completely submerged in the liquid.

Filling the test device wells with serum dilution can be carried out in two ways. If only a little serum is available the test devices can be filled using the robot arm mentioned in the previous example and when sufficient serum is available the test devices can be submerged in the serum and subsequently be wiped so that all the wells are simultaneously filled.

Incubation of the test devices took place during the night at 4°C in air saturated with water, after which the test devices were washed three times with 0.05% Tween® 80/PBS in order to remove antibodies that had not been bound. The test devices according to the invention that had been incubated with serum were subsequently incubated for 1 hour at 37°C with an antibody conjugated peroxidase enzyme (1/1000 solution in SuperQ®) by submerging the test devices in a solution comprising said enzyme. After this the test devices were rinsed with PBS, 3x 10 min. The presence of the second antibody was demonstrated with the substrate liquid ABTS (2,2'-Azine-di[3-ethyl benzthiazoline sulfonate (6)]). In this instance it is also possible to use the two aforementioned methods to fill the wells, either using the robot arm or submerging the devices in the substrate.

## Claims (Claims for the following Contracting State(s): AT, BE, DK, ES, GR, IT, LU, NL, SE, IE)

1. Test device comprising a plate containing a multiplicity of wells, **characterised** in that the wells have a volume within the range of 0.1-20 µl, and that the diameter of the wells is 1.0-4.0 mm, preferably 1.0-2.0 mm.

2. Test device according to claim 1, **characterised** in that the wells have a volume within the range of 0.1-5 µl.

3. Test device according to any of the preceding claims, **characterised** in that the ratio between the depth of the wells and the diameter thereof is less than 1:1, preferably less than 2:3.

4. Test device according to any of the preceding claims, **characterised** in that a vertical cross section of the wells is essentially U-shaped, the transition between legs and base of the U being gradual.

5. Test device according to any of the preceding claims, **characterised** in that the wells are separated by material barriers with a width between 1.0-5.0 mm and preferably between 1.0 and 2.0 mm.

6. Test device according to any of the preceding claims, **characterised** in that the plate contains 5-20 wells per cm², preferably 10-15 wells per cm².

7. Test device according to any of the preceding claims, **characterised** in that the wells of the plate are composed of material to which biochemical molecules, such as hormones and polysaccharides, and preferably peptides and proteins, are able to adhere.

8. Test device according to any of the preceding claims, **characterised** in that the bottom of the wells is sufficiently transparent for carrying out photometric analysis.

9. Method for carrying out a (bio- and/or immuno)chemical test, in particular a method comprising a rinsing step such as an ELISA or pepscan, **characterised** in that a test device according to any of claims 1-8 is used.

10. Method according to claim 9, **characterised** in that the test is carried out using sample amounts of between 0.1 and 5 µl.

11. Metering device suitable for simultaneously introducing equal volumes of reagent into different wells of a test device comprising a microtitre plate containing a multiplicity of well, **characterised** in that said device is provided with projections which have dimensions and mutual spacings such that individual projections can simultaneously be placed in or above the wells of a test device according to claims 1-8, intended to be provided with reagent, said projections optionally being detachable.

12. Metering device according to claim 11, **characterised** in that the position of the projections is such that said position essentially corresponds to the position of the wells in the test device according to any of claims 1-8.

13. A metering device according to claim 11 or 12, said projections being hollow.

14. Metering device according to any of claims 11-13, characterised in that the projections are closed at the bottom.

15. Method for carrying out a (bio- and/or immuno)chemical test using a metering device according to any of claims 11-14, in which method
a) the projections of the metering device are provided with reagent, in such a way that essentially equal volumes of reagent are present on or in the individual projections of the metering device, and
b) the metering device is then positioned in or above the wells of the test device according to any of claims 1-8, said wells to be provided with reagent, each individual projection or each group of projections being located in or above a well at the same time, and
c) essentially equal volumes of reagent are introduced into the individual wells of the test device, said wells to be provided with reagent.

16. Method for carrying out a (bio- and/or immuno)chemical test according to Claim 15, in which method, in step a), the projections of the metering device are simultaneously provided with reagent by immersing the projections in reagent.

17. Kit which comprises at least a test device according to any of claims 1-8 and at least a metering device according to any of claims 11-14.

## Claims (Claims for the following Contracting State(s): CH, DE, FR, GB, LI)

1. Test device comprising a microtitre plate containing a multiplicity of wells, **characterised** in that the wells have a volume within the range of 0.1-5 µl.

2. Test device according to claim 1, **characterised** in that the wells have a diameter within the range of 1.0 - 4.0 mm, preferably 1.0 - 2.0 mm.

3. Test device according to any of the preceding claims, **characterised** in that the ratio between the depth of the wells and the diameter thereof is less than 1:1, preferably less than 2:3.

4. Test device according to any of the preceding claims, **characterised** in that a vertical cross section of the wells is essentially U-shaped, the transition between legs and base of the U being gradual.

5. Test device according to any of the preceding claims, **characterised** in that the wells are separated by material barriers with a width between 1.0-5.0 mm and preferably between 1.0 and 2.0 mm.

6. Test device according to any of the preceding claims, **characterised** in that the plate contains 5-20 wells per cm², preferably 10-15 wells per cm²**.**

7. Test device according to any of the preceding claims, **characterised** in that the wells of the plate are composed of material to which biochemical molecules, such as hormones and polysaccharides, and preferably peptides and proteins, are able to adhere.

8. Test device according to any of the preceding claims, **characterised** in that the bottom of the wells is sufficiently transparent for carrying out photometric analysis.

9. Method for carrying out a (bio- and/or immuno)chemical test, in particular a method comprising a rinsing step such as an ELISA or pepscan, **characterised** in that a test device according to any of claims 1-8 is used.

10. Method according to claim 9, **characterised** in that the test is carried out using sample amounts of between 0.1 and 5 µl.

11. Metering device suitable for simultaneously introducing equal volumes of reagent into different wells of a test device comprising a microtitre plate containing a multiplicity of wells, **characterised** in that said device is provided with projections which have dimensions and mutual spacings such that individual projections can simultaneously be placed in or above the wells of a test device according to claims 1-8, intended to be provided with reagent with the proviso the device does not comprise 864 projections.

12. A metering device according to claim 11, said projections being detachable.

13. A metering device according to claim 11 or 12, said projections being hollow.

14. Metering device according to any of claims 11 - 13, **characterised** in that the position of the projections is such that said position essentially corresponds to the position of the wells in the test device according to any of claims 1-8.

15. Metering device according to any of claims 11-14, characterised in that the projections are closed at the bottom.

16. Method for carrying out a (bio- and/or immuno)chemical test using a metering device according to any of claims 11-15, in which method
a) the projections of the metering device are provided with reagent, in such a way that essentially equal volumes of reagent are present on or in the individual projections of the metering device, and
b) the metering device is then positioned in or above the wells of the test device according to any of claims 1-8, said wells to be provided with reagent, each individual projection or each group of projections being located in or above a well at the same time, and
c) essentially equal volumes of reagent are introduced into the individual wells of the test device, said wells to be provided with reagent.

17. Method for carrying out a (bio- and/or immuno)chemical test according to Claim 16, in which method, in step a), the projections of the metering device are simultaneously provided with reagent by immersing the projections in reagent.

18. Kit which comprises at least a test device according to any of claims 1-8 and at least a metering device according to any of claims 11-15.

## Patentansprüche (Patentansprüche für folgende(n) Vertragsstaat(en): AT, BE, DK, ES, GR, IT, LU, NL, SE, IE)

1. Testvorrichtung, umfassend eine Platte mit einer Vielzahl von Vertiefungen, dadurch gekennzeichnet, daß die Vertiefungen ein Volumen aufweisen innerhalb des Bereiches von 0,1 bis 20 µl, und daß der Durchmesser der Vertiefungen 1,0 bis 4,0 mm, bevorzugt 1,0 bis 2,0 mm, beträgt.

2. Testvorrichtung nach Anspruch 1, dadurch gekennzeichnet, daß die Vertiefungen ein Volumen innerhalb des Bereiches von 0,1 bis 5 µl aufweisen.

3. Testvorrichtung nach einem der vorangegangenen Ansprüche, dadurch gekennzeichnet, daß das Verhältnis zwischen der Tiefe der Vertiefungen und dem Durchmesser davon kleiner ist als 1 : 1, bevorzugt kleiner als 2 : 3.

4. Testvorrichtung nach einem der vorangegangenen Ansprüche, dadurch gekennzeichnet, daß ein vertikaler Querschnitt der Vertiefungen im wesentlichen U-förmig ist, wobei der Übergang zwischen Schenkeln und Basis des U graduell erfolgt.

5. Testvorrichtung nach einem der vorangegangenen Ansprüche, dadurch gekennzeichnet, daß die Vertiefungen getrennt sind durch Materialbarrieren mit einer Breite zwischen 1,0 und 5 mm und bevorzugt zwischen 1,0 und 2,0 mm.

6. Testvorrichtung nach einem der vorangegangenen Ansprüche, dadurch gekennzeichnet, daß die Platte 5 bis 20 Vertiefungen pro cm², bevorzugt 10 bis 15 Vertiefungen pro cm² enthält.

7. Testvorrichtung nach einem der vorangegangenen Ansprüche, dadurch gekennzeichnet, daß die Vertiefungen der Platte gebildet sind aus Material, an welchem biochemische Moleküle wie z.B. Hormone und Polysaccharide, und bevorzugt Peptide und Proteine, anhaften können.

8. Testvorrichtung nach einem der vorangegangenen Ansprüche, dadurch gekennzeichnet, daß der Boden der Vertiefungen ausreichend transparent ist, um photometrische Analysen durchzuführen.

9. Verfahren zum Durchführen eines (bio- und/oder immun)-chemischen Testes, insbesondere ein Verfahren, umfassend einen Spülschritt, wie z.B. ein ELISA oder Pepscan, dadurch gekennzeichnet, daß eine Testvorrichtung gemäß einem der Ansprüche 1 bis 8 verwendet wird.

10. Verfahren nach Anspruch 9, dadurch gekennzeichnet, daß der Test durchgeführt wird unter Verwendung von Probenmengen von zwischen 0,1 und 5 µl.

11. Meßvorrichtung, welche geeignet ist zum gleichzeitigen Einführen gleicher Volumina von Reagenz in verschiedene Vertiefungen einer Testvorrichtung, umfassend eine Mikrotitrierplatte, welche eine Vielzahl von Vertiefungen enthält, dadurch gekennzeichnet, daß die Vorrichtung bereitgestellt ist mit Vorsprüngen, welche Abmessungen und gegenseitige Beabstandungen aufweisen, welche derart sind, daß einzelne Vorsprünge gleichzeitig in oder über den Vertiefungen einer Testvorrichtung gemäß einem der Ansprüche 1 bis 8 angeordnet werden können, beabsichtigt, um mit Reagenzien versehen zu werden, wobei die Vorsprünge optional lösbar sind.

12. Meßvorrichtung nach Anspruch 11, dadurch gekennzeichnet, daß die Position der Vorsprünge derart ist, daß die Position im wesentlichen der Position der Vertiefungen in der Testvorrichtung nach einem der Ansprüche 1 bis 8 entspricht.

13. Meßvorrichtung nach Anspruch 11 oder 12, bei welcher die Vorsprünge hohl sind.

14. Meßvorrichtung nach einem der Ansprüche 11 bis 13, dadurch gekennzeichnet, daß die Vorsprünge an dem Boden geschlossen sind.

15. Verfahren zum Durchführen eines (bio- und/oder immun)-chemischen Testes unter Verwendung einer Meßvorrichtung nach einem der Ansprüche 11 bis 14, in welchem Verfahren
(a) die Vorsprünge der Meßvorrichtung bereitgestellt sind mit Reagenz, in solch einer Weise, daß im wesentlichen gleiche Volumina von Reagenz an oder in den einzelnen Vorsprüngen der Meßvorrichtung vorhanden sind, und
(b) die Meßvorrichtung nachfolgend in oder über den Vertiefungen der Testvorrichtung gemäß einem der Ansprüche 1 bis 8 angeordnet wird, wobei die Vertiefungen mit Reagenz zu versehen sind, wobei jeder einzelne Vorsprung oder jede Gruppe von Vorsprüngen in oder über einer Vertiefung gleichzeitig angeordnet ist, und
(c) im wesentlichen gleiche Volumina an Reagenz in die einzelnen Vertiefungen der Testvorrichtung eingeführt werden, wobei die Vertiefungen mit Reagenz zu versehen sind.

16. Verfahren zum Durchführen eines (bio- und/oder immun)-chemischen Testes nach Anspruch 15, in welchem Verfahren im Schritt (a) die Vorsprünge der Meßvorrichtung gleichzeitig bereitgestellt sind mit Reagenz durch Eintauchen der Vorsprünge in Reagenz.

17. Kitt, umfassend zumindest eine Testvorrichtung nach einem der Ansprüche 1 bis 8 und zumindest eine Meßvorrichtung nach einem der Ansprüche 11 bis 14.

## Patentansprüche (Patentansprüche für folgende(n) Vertragsstaat(en): CH, DE, FR, GB, LI)

1. Testvorrichtung, umfassend eine Mikrotitrierplatte, welche eine Vielzahl von Vertiefungen enthält, dadurch gekennzeichnet, daß die Vertiefungen ein Volumen innerhalb des Bereiches von 0,1 bis 5 µl aufweisen.

2. Testvorrichtung nach Anspruch 1, dadurch gekennzeichnet, daß die Vertiefungen einen Durchmesser innerhalb des Bereiches von 1,0 bis 4,0 mm bevorzugt von 1,0 bis 2,0 mm, aufweisen.

3. Testvorrichtung nach einem der vorangegangenen Ansprüche, dadurch gekennzeichnet, daß das Verhältnis zwischen der Tiefe der Vertiefungen und dem Durchmesser davon kleiner ist als 1 : 1, bevorzugt kleiner als 2 : 3.

4. Testvorrichtung nach einem der vorangegangenen Ansprüche, dadurch gekennzeichnet, daß ein vertikaler Querschnitt der Vertiefungen im wesentlichen U-förmig ist, wobei der Übergang zwischen Schenkein und Basis des U graduell erfolgt.

5. Testvorrichtung nach einem der vorangegangenen Ansprüche, dadurch gekennzeichnet, daß die Vertiefungen getrennt sind durch Materialbarrieren mit einer Breite zwischen 1,0 und 5 mm und bevorzugt zwischen 1,0 und 2,0 mm.

6. Testvorrichtung nach einem der vorangegangenen Ansprüche, dadurch gekennzeichnet, daß die Platte 5 bis 20 Vertiefungen pro cm², bevorzugt 10 bis 15 Vertiefungen pro cm² enthält.

7. Testvorrichtung nach einem der vorangegangenen Ansprüche, dadurch gekennzeichnet, daß die Vertiefungen der Platte gebildet sind aus Material, an welchem biochemische Moleküle wie z.B. Hormone und Polysaccharide, und bevorzugt Peptide und Proteine, anhaften können.

8. Testvorrichtung nach einem der vorangegangenen Ansprüche, dadurch gekennzeichnet, daß der Boden der Vertiefungen ausreichend transparent ist, um photometrische Analysen durchzuführen.

9. Verfahren zum Durchführen eines (bio- und/oder immun)-chemischen Testes, insbesondere ein Verfahren, umfassend einen Spülschritt, wie z.B. ein ELISA oder Pepscan, dadurch gekennzeichnet, daß eine Testvorrichtung gemäß einem der Ansprüche 1 bis 8 verwendet wird.

10. Verfahren nach Anspruch 9, dadurch gekennzeichnet, daß der Test durchgeführt wird unter Verwendung von Probenmengen von zwischen 0,1 und 5 µl.

11. Meßvorrichtung, welche geeignet ist zum gleichzeitigen Einführen gleicher Volumina von Reagenz in verschiedene Vertiefungen einer Testvorrichtung, umfassend eine Mikrotitrierplatte, welche eine Vielzahl von Vertiefungen enthält, dadurch gekennzeichnet, daß die Vorrichtung bereitgestellt ist mit Vorsprüngen, welche Abmessungen und gegenseitige Beabstandungen aufweisen, welche derart sind, daß einzelne Vorsprünge gleichzeitig in oder über den Vertiefungen einer Testvorrichtung gemäß einem der Ansprüche 1 bis 8 angeordnet werden können, beabsichtigt, um mit Reagenzien versehen zu werden unter Voraussetzung, daß die Vorrichtung nicht 864 Vorsprünge enthält.

12. Meßvorrichtung nach Anspruch 11, bei welcher die Vorsprünge lösbar sind.

13. Meßvorrichtung nach Anspruch 11 oder 12, bei welcher die Vorsprünge hohl sind.

14. Meßvorrichtung nach einem der Ansprüche 11 bis 13, dadurch gekennzeichnet, daß die Position der Vorsprünge derart ist, daß die Position im wesentlichen der Position der Vertiefungen in der Testvorrichtung nach einem der Ansprüche 1 bis 8 entspricht.

15. Meßvorrichtung nach einem der Ansprüche 11 bis 14, dadurch gekennzeichnet, daß die Vorsprünge an dem Boden geschlossen sind.

16. Verfahren zum Durchführen eines (bio- und/oder immun)-chemischen Testes unter Verwendung einer Meßvorrichtung nach einem der Ansprüche 11 bis 15, in welchem Verfahren
(a) die Vorsprünge der Meßvorrichtung bereitgestellt sind mit Reagenz, in solch einer Weise, daß im wesentlichen gleiche Volumina von Reagenz an oder in den einzelnen Vorsprüngen der Meßvorrichtung vorhanden sind, und
(b) die Meßvorrichtung nachfolgend in oder über den Vertiefungen der Testvorrichtung gemäß einem der Ansprüche 1 bis 8 angeordnet wird, wobei die Vertiefungen mit Reagenz zu versehen sind, wobei jeder einzelne Vorsprung oder jede Gruppe von Vorsprüngen in oder über einer Vertiefung gleichzeitig angeordnet ist, und
(c) im wesentlichen gleiche Volumina an Reagenz in die einzelnen Vertiefungen der Testvorrichtung eingeführt werden, wobei die Vertiefungen mit Reagenz zu versehen sind.

17. Verfahren zum Durchführen eines (bio- und/oder immun)-chemischen Testes nach Anspruch 16, in welchem Verfahren im Schritt (a) die Vorsprünge der Meßvorrichtung gleichzeitig bereitgestellt sind mit Reagenz durch Eintauchen der Vorsprünge in Reagenz.

18. Kitt, umfassend zumindest eine Testvorrichtung nach einem der Ansprüche 1 bis 8 und zumindest eine Meßvorrichtung nach einem der Ansprüche 11 bis 15.

## Revendications (Revendications pour l'(les) Etat(s) contractant(s) suivant(s): AT, BE, DK, ES, GR, IT, LU, NL, SE, IE)

1. Dispositif d'analyse comprenant une plaque contenant une multiplicité de puits, caractérisé en ce que les puits ont un volume dans la gamme de 0,1-20 µl, et en ce que le diamètre des puits est de 1,0-4,0 mm, de préférence 1,0-2,0 mm.

2. Dispositif d'analyse selon la revendication 1, caractérisé en ce que les puits ont un volume dans la gamme de 0,1-5 µl.

3. Dispositif d'analyse selon l'une quelconque des revendications qui précèdent, caractérisé en ce que le rapport entre la profondeur des puits et leur diamètre est inférieur à 1:1, de préférence inférieur à 2:3.

4. Dispositif d'analyse selon l'une quelconque des revendications qui précèdent, caractérisé en ce qu'une section en coupe verticale des puits est essentiellement en forme de U, la transition entre les branches et la base du U étant progressive.

5. Dispositif d'analyse selon l'une quelconque des revendications précédentes, caractérisé en ce que les puits sont séparés par des barrières matérielles d'une largeur comprise entre 1,0-5,0 mm et, de préférence, entre 1,0 et 2,0 mm.

6. Dispositif d'analyse selon l'une quelconque des revendications précédentes, caractérisé en ce que la plaque contient de 5 à 20 puits par cm², de préférence de 10 à 15 puits par cm².

7. Dispositif d'analyse selon l'une quelconque des revendications précédentes, caractérisé en ce que les puits de la plaque sont en un matériau auquel des molécules biochimiques, telles que des hormones et des polysaccharides, et de préférence des peptides et des protéines, peuvent adhérer.

8. Dispositif d'analyse selon l'une quelconque des revendications précédentes, caractérisé en ce que le fond des puits est suffisamment transparent pour pouvoir procéder à une analyse photométrique.

9. Procédé pour effectuer une analyse (bio et/ou immuno)chimique, en particulier procédé comprenant une étape de rinçage tel qu'un dosage par la méthode ELISA ou une scintigraphie peptidique, caractérisé en ce qu'un dispositif d'analyse selon l'une quelconque des revendications 1-8 est utilisé.

10. Procédé selon la revendication 9, caractérisé en ce que l'analyse est effectuée en utilisant des quantités d'échantillons comprises entre 0,1 et 5 µl.

11. Dispositif de dosage approprié pour introduire simultanément des volumes égaux de réactif dans différents puits d'un dispositif d'analyse comprenant une plaque de microtitrage contenant une multiplicité de puits, caractérisé en ce que ledit dispositif est pourvu de saillies qui ont des dimensions et des espacements mutuels tels que les saillies individuelles peuvent être simultanément positionnées dans ou au-dessus des puits d'un dispositif d'analyse selon les revendications 1-8, destinés à être garnis de réactif, lesdites saillies étant éventuellement amovibles.

12. Dispositif de dosage selon la revendication 11, caractérisé en ce que la position des saillies est telle que ladite position correspond essentiellement à la position des puits dans le dispositif d'analyse selon l'une quelconque des revendications 1-8.

13. Dispositif de dosage selon la revendication 11 ou 12, caractérisé en ce que lesdites projections sont creuses.

14. Dispositif de dosage selon l'une quelconque des revendications 11 à 13, caractérisé en ce que les saillies sont fermées au niveau de leur partie inférieure.

15. Procédé pour effectuer une analyse (bio et/ou immuno)chimique en utilisant un dispositif de dosage selon l'une quelconque des revendications 11-14, procédé dans lequel
a) les saillies du dispositif de dosage sont garnies de réactif, de manière que des volumes essentiellement égaux de réactif soient présents sur ou dans les saillies individuelles du dispositif de dosage, et
b) le dispositif de dosage est ensuite positionné dans ou au-dessus des puits du dispositif d'analyse selon l'une quelconque des revendications 1-8, lesdits puits étant destinés à être garnis de réactif, chaque saillie individuelle ou chaque groupe de saillies étant positionné simultanément dans ou au-dessus d'un puits, et
c) des volumes essentiellement égaux de réactif sont introduits dans les puits individuels du dispositif d'analyse, lesdits puits étant destinés à être garnis de réactif.

16. Procédé pour effectuer une analyse (bio et/ou immuno)chimique selon la revendication 15, procédé dans lequel, dans l'étape a), les saillies du dispositif de dosage sont simultanément garnies de réactif en immergeant les saillies dans le réactif.

17. Trousse qui comprend au moins un dispositif d'analyse selon l'une quelconque des revendications 1-8 et au moins un dispositif de dosage selon l'une quelconque des revendications 11-14.

## Revendications (Revendications pour l'(les) Etat(s) contractant(s) suivant(s): CH, DE, FR, GB, LI)

1. Dispositif d'analyse comprenant une plaque de microtitrage contenant une multiplicité de puits, caractérisé en ce que les puits ont un volume dans la gamme de 0,1-5 µl.

2. Dispositif d'analyse selon la revendication 1, caractérisé en ce que les puits ont un diamètre de 1,0-4,0 mm, de préférence de 1,0-2,0 mm.

3. Dispositif d'analyse selon l'une quelconque des revendications précédentes, caractérisé en ce que le rapport entre la profondeur des puits et leur diamètre est inférieur à 1:1, de préférence inférieur à 2:3.

4. Dispositif d'analyse selon l'une quelconque des revendications précédentes, caractérisé en ce qu'une section en coupe verticale des puits est essentiellement en forme de U, la transition entre les branches et la base du U étant progressive.

5. Dispositif d'analyse selon l'une quelconque des revendications précédentes, caractérisé en ce que les puits sont séparés par des barrières matérielles d'une largeur comprise entre 1,0-5,0 mm et, de préférence, entre 1,0 et 2,0 mm.

6. Dispositif d'analyse selon l'une quelconque des revendications qui précèdent, caractérisé en ce que la plaque contient de 5 à 20 puits par cm², de préférence de 10 à 15 puits par cm².

7. Dispositif d'analyse selon l'une quelconque des revendications précédentes, caractérisé en ce que les puits de la plaque sont en un matériau auquel des molécules biochimiques, telles que des hormones et des polysaccharides, et de préférence des peptides et des protéines, peuvent adhérer.

8. Dispositif d'analyse selon l'une quelconque des revendications précédentes, caractérisé en ce que le fond des puits est suffisamment transparent pour pouvoir procéder à une analyse photométrique.

9. Procédé pour effectuer une analyse (bio et/ou immuno)chimique, en particulier procédé comprenant une étape de rinçage tel qu'une méthode ELISA ou une méthode PEPSCAN, caractérisé en ce qu'un dispositif d'analyse selon l'une quelconque des revendications 1-8 est utilisé.

10. Procédé selon la revendication 9, caractérisé en ce que l'analyse est effectuée en utilisant des quantités d'échantillons comprises entre 0,1 et 5 µl.

11. Dispositif de dosage approprié pour introduire simultanément des volumes égaux de réactif dans différents puits d'un dispositif d'analyse comprenant une plaque de microtitrage contenant une multiplicité de puits, caractérisé en ce que ledit dispositif est pourvu de saillies qui ont des dimensions et des espacements mutuels tels que les saillies individuelles peuvent être simultanément positionnées dans ou au-dessus des puits d'un dispositif d'analyse selon les revendications 1-8, destinés à être garnis de réactif, à condition que le dispositif ne comprenne pas 864 saillies.

12. Dispositif de dosage selon la revendication 11, dans lequel lesdites saillies sont amovibles.

13. Dispositif de dosage selon la revendication 11 ou 12, dans lequel lesdites saillies sont creuses.

14. Dispositif de dosage selon l'une quelconque des revendications 11 à 13, caractérisé en ce que la position des saillies est telle que ladite position correspond essentiellement à la position des puits dans le dispositif d'analyse selon l'une quelconque des revendications 1-8.

15. Dispositif de dosage selon l'une quelconque des revendications 11 à 14, caractérisé en ce que les saillies sont fermées au niveau de leur partie inférieure.

16. Procédé pour effectuer une analyse (bio et/ou immuno)chimique en utilisant un dispositif de dosage selon l'une quelconque des revendications 11-15, procédé dans lequel
a) les saillies du dispositif de dosage sont garnies de réactif, de manière que des volumes essentiellement égaux de réactif soient présents sur ou dans les saillies individuelles du dispositif de dosage, et
b) le dispositif de dosage est ensuite positionné dans ou au-dessus des puits du dispositif d'analyse selon l'une quelconque des revendications 1-8, lesdits puits étant destinés à être garnis de réactif, chaque saillie individuelle ou chaque groupe de saillies étant positionné simultanément dans ou au-dessus d'un puits, et
c) des volumes essentiellement égaux de réactif sont introduits dans les puits individuels du dispositif d'analyse, lesdits puits étant destinés à être garnis de réactif.

17. Procédé pour effectuer une analyse (bio et/ou immuno)chimique selon la revendication 16, procédé dans lequel, dans l'étape a), les saillies du dispositif de dosage sont simultanément garnies de réactif en immergeant les saillies dans le réactif.

18. Trousse qui comprend au moins un dispositif d'analyse selon l'une quelconque des revendications 1-8 et au moins un dispositif de dosage selon l'une quelconque des revendications 11-15.
